(19) Europäisches Patentamt
European Patent Office
Office européen des brevets

(11) EP 1 303 278 B1

(12) EUROPÄISCHE PATENTSCHRIFT

(45) Veröffentlichungstag und Bekanntmachung des Hinweises auf die Patenterteilung:
**05.11.2008 Patentblatt 2008/45**

(21) Anmeldenummer: **01976150.1**

(22) Anmeldetag: **24.08.2001**

(51) Int Cl.:
***A61K 31/48*** (2006.01) ***A61P 25/14*** (2006.01)
***A61P 25/16*** (2006.01) ***A61K 31/45*** (2006.01)
***A61K 31/13*** (2006.01) ***A61K 9/00*** (2006.01)
***A61K 9/20*** (2006.01) ***A61K 9/70*** (2006.01)

(86) Internationale Anmeldenummer:
**PCT/EP2001/009826**

(87) Internationale Veröffentlichungsnummer:
**WO 2002/034267 (02.05.2002 Gazette 2002/18)**

(54) **KOMBINATION EINES TRANSDERMALEN THERAPEUTISCHEN SYSTEMS UND EINER ORALEN UND/ODER PARENTERALEN ZUBEREITUNG ENTHALTEND DOPAMINAGONISTEN ZUR BEHANDLUNG DOPAMINERGER ERKRANKUNGEN**

COMBINATION OF A TRANSDERMAL THERAPEUTIC SYSTEM AND AN ORAL AND/OR PARENTERAL PREPARATION CONTAINING DOPAMINE AGONISTS FOR THE TREATMENT OF DOPAMINERGIC DISEASE STATES

COMBINAISON D'UN SYSTEME THERAPEUTIQUE TRANSDERMAL ET D'UNE PREPARATION ORALE ET/OU PARENTERALE CONTENANT DES AGONISTES DOPAMINERGIQUES POUR TRAITER DES MALADIES EN RELATION AVEC LA DOPAMINE

(84) Benannte Vertragsstaaten:
**AT BE CH CY DE DK ES FI FR GB GR IE IT LI LU MC NL PT SE TR**

(30) Priorität: **20.10.2000 DE 10053397**

(43) Veröffentlichungstag der Anmeldung:
**23.04.2003 Patentblatt 2003/17**

(73) Patentinhaber: **Axxonis Pharma AG**
**10963 Berlin (DE)**

(72) Erfinder:
- **HOROWSKI, Reinhard**
  **14129 Berlin (DE)**
- **TACK, Johannes**
  **13595 Berlin (DE)**

(74) Vertreter: **Wablat, Wolfgang**
**Patentanwalt**
**Dr. Dr. W. Wablat**
**Potsdamer Chaussee 48**
**14129 Berlin (DE)**

(56) Entgegenhaltungen:
**EP-A- 0 458 640** **WO-A-01/28555**
**DE-A- 4 240 798** **US-A- 5 597 832**
**US-A- 5 650 420**

- **DEN DAAS ET AL: "Transdermal administration of the dopamine agonist N-0437 and seven ester prodrugs: comparison with oral administration in the 6-OHDA turning model" NAUNYN-SCHMIEDEBERG'S ARCHIVES OF PHARMACOLOGY, SPRINGER, BERLIN, DE, Bd. 342, Nr. 6, Dezember 1990 (1990-12), Seiten 655-659, XP002110534 ISSN: 0028-1298**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 303 278 B1

## Beschreibung

Die Erfindung betrifft die Verwendung eines Mittels umfassend ein transdermales therapeutisches System (TTS) enthaltend als Wirkstoff einen Dopaminagonisten zur Behandlung von dopaminerg behandelbaren Erkrankungen im Rahmen eines besonderen Behandlungsplans.

Erkrankungen, bei denen eine dopaminerge Therapie angezeigt ist, wie beispielsweise Morbus Parkinson, sind schwere chronische und behindernde Erkrankungen, unter welchen oft ältere und polymorbide Patienten leiden. In der Praxis erfolgt eine Behandlung durch orale Gabe einer Kombination dopaminerger Substanzen. Diese umfasst im allgemeinen verschiedene Formulierungen von Levodopa (schnell anflutend, normale oder langsame Freisetzung), Levodopa-Verstärker beispielsweise als Basis Decarboxylase-Inhibitoren und gegebenenfalls COMT-*Inhibitoren* oder MAO-B-Inhibitoren und verschiedene Dopaminagonisten, wie beispielsweise Bromocriptin, Lisurid, Cabergolin, Pergolid, Ropinirol, Pramipexol , sowie weiterhin Amantadine und gelegentlich anticholinerge Wirkstoffe. Aus verschiedenen Gründen hat Levodopa, welches ein sehr schnell wirkender Wirkstoff ist, eine schwer beherrschbare Pharmakokinetik und auch Dopaminagonisten erlauben häufig keine sicheren Prognosen hinsichtlich der biologischen Verfügbarkeit und folglich der effektiven Wirkung. All diese Wirkstoffe können aus pharmakologischen und pharmakokinetischen Gründen zudem miteinander Wechselwirkungen zeigen, und dies zusätzlich zu Interaktionen mit anderen Wirkstoffen bzw.

Medikamenten, welche insbesondere ältere multimorbide Patienten sehr häufig benötigen.

Je nach Stadium der Erkrankung und momentanem Zustand des Patienten kann eine eher kontinuierliche oder diskontinuierliche dopaminerge Stimulation erforderlich sein. Ein stabiler dopaminerger Wirkstoffpegel über den ganzen Tag ist meist eine gute Basis. Patienten berichten jedoch oft, dass insbesondere morgens oder zu bestimmten Zeiten im Tagesablauf eine Einnahme eines schnell wirkenden dopaminergen Wirkstoffes erforderlich ist, um akute motorische Störungen, schwere und schmerzvolle Dystonien etc., zu überwinden ("kick"). In extremen Fällen sind plötzliche "off" Situationen der motorischen Leistung und Akinese (manchmal vorhersehbar am frühen Morgen oder frühen Nachmittag, oft aber ganz plötzlich und unerwartet) nur mit injizierbaren Wirkstoffen, wie Apomorphin, beherrschbar. Auf der anderen Seite können starke und rasche Wirkungsanstiege störende Nebenwirkungen (z.B. Nausea, Emesis, orthostatische Hypotension, sogenannte Schlafattacken) hervorrufen. Auch Überdosen, bedingt durch ein enges therapeutisches Zeitfenster all dieser dopaminergen Wirkstoffe, können in schwerer Dyskinesie, Dystonie oder, insbesondere bei älteren Patienten, Psychosen resultieren. Das letztere schwere Problem hängt insbesondere zusammen mit hohen Plasmakonzentrationen des Wirkstoffes in der Nacht, die bekanntermaßen normale Schlafmuster zerstören und normalen REM-Schlaf verhindern (mit REM-rebound zur Tageszeit als erstes Anzeichen einer Psychose).

Aufgrund der vorstehenden Zusammenhänge wird eine dopaminerge Therapie in der Praxis mit sehr geringen Dosen eines oder mehrerer Wirkstoffe begonnen, gefolgt von subsequenter Erhöhung der Dosis, beispielsweise wöchentlich, bis Nebenwirkungen die biologische Verfügbarkeit anzeigen. Nach einer anschließenden eher willkürlichen Reduktion der Dosierung oder Stabilisierung wird mit der Gabe des nächsten Wirkstoffes begonnen und dieser dann auf der vorstehenden entsprechende Weise individuell eingestellt bzw. dosiert ("titriert"). Im Ergebnis variieren Behandlungspläne und insbesondere die Dosierungen in erheblichem Maße, abhängig von der Schwere der Erkrankung und der individuellen Konstitution des Patienten und seinem Metabolisierungstyp. Meist werden 3 oder mehr verschiedene Wirkstoffe oral verabreicht. Ein typischer Patient beginnt beispielsweise am Morgen mit schnell wirkendem Levodopa, gefolgt von einer Gabe eines MAO-B-Inhibitors und, verteilt über den Tag, der vier- oder öftermaligen Einnahme normal wirkenden Levodopas in Kombination mit einem Dopamin-Agonisten, und, letztendlich, einer langsam wirkenden Zubereitung enthaltend Levodopa (oder eines niedrig dosierten, lang wirkenden Dopamin-Agonisten) zur Schlafensgehzeit, womit eine ausreichende Bewegungsfähigkeit während des Schlafes und folglich ein hoher Erholungswert gewährleistet werden soll.

Ein solch komplizierter Behandlungsplan, welcher eher die Regel als die Ausnahme bildet, weist insbesondere bei älteren Patienten eine schlechte Verträglichkeit auf und ist zudem eher unstabil und empfindlich gegen Wechselwirkungen mit anderen Faktoren, wie der Gabe anderer Wirkstoffe oder infektionsbedingten Erkrankungen sowie auch Dehydrierung durch zu geringe Flüssigkeitsaufnahme oder zu starken Flüssigkeitsverlust oder Leber- oder Nierenfunktionsstörungen. Dies ist sowohl für die behandelnden Ärzte als auch für die Patienten aus offensichtlichen Gründen unbefriedigend. Daher muss die Einstellung der Patienten in Hinblick auf Nebenwirkungen meist in mehr oder weniger spezialisierten Krankenhäusern stationär über mehrere Wochen erfolgen.

Der Erfindung liegt das technische Problem zugrunde, ein Mittel sowie einen Behandlungsplan zur Behandlung von dopaminerg behandelbaren Erkrankungen anzugeben, wobei störende Nebenwirkungen vermieden, zumindest jedoch reduziert sind, und wobei der Wirkstoff kontrolliert anflutet und in Anflutung, Plasmakonzentration und Wirkdauer gut steuerbar ist.

Zur Lösung dieses technischen Problems lehrt die Erfindung die Verwendung eines Ergolin-Derivats gemäss Formel I oder dessen physiologisch verträglichem Salz,

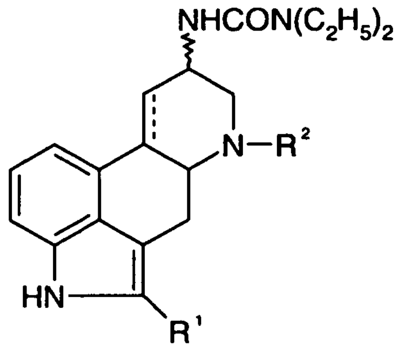

**Formel I**

worin --------- eine Einfachbindung oder eine Doppelbindung ist, worin R1 ein H-Atom oder ein Halogenatom, insbesondere ein Bromatom ist, und worin R2 C1-4-Alkyl ist, zur Herstellung eines Mittels zur Behandlung von Erkrankungen aus der Gruppe bestehend aus Morbus Parkinson, Parkinsonismus, Restless-Legs-Syndrom und Störungen des dopaminergen Systems, **dadurch gekennzeichnet, dass**
das Mittel in mindestens zwei räumlich getrennten Formen vorliegt, wovon eine das Ergolin-Derivat in einem transdermalen therapeutischen System (TTS) enthält, und eine oder mehrere weitere Formen dasselbe Ergolin-Derivat in einer zur oralen und/oder parenteralen Gabe hergerichteten Zubereitung enthalten,
und dass das TTS für eine kontinuierliche Applikation vorgesehen ist und die zur oralen und/oder parenteralen Gabe hergerichtete Zubereitung für eine diskontinuierliche Gabe innerhalb der Applikationsdauer des TTS vorgesehen ist.

Der Begriff der kontinuierliche Applikation meint, dass ein neues TTS appliziert wird, bevor die Plasmakonzentration aufgrund des vorherigen TTS aufbrauchsbedingt in störender Weise sinkt, beispielsweise unterhalb des 0,25-fachen der maximalen Plasmakonzentration.

Die Erfindung beruht auf der überraschenden Erkenntnis, dass dopaminerg behandelbare Erkrankungen, insbesondere Morbus Parkinson, sich mit einem einzigen dopaminergen Wirkstoff, der hocheffektiv ist und eine kurze Halbwertszeit im Plasma aufweist, besser behandeln lässt, wenn die erfindungsgemäße Kombination der Zubereitungen gegebenenfalls in Verbindung mit einem der erfindungsgemäßen Behandlungspläne realisiert wird. Wesentlich ist also, dass während der Behandlungsdauer kein anderer als der Wirkstoff des TTS zur Behandlung dopaminerger Störungen eingesetzt wird. Eine dauernde bzw. kontinuierliche dopaminerge Stimulation wird mit dem TTS erreicht. Hierdurch werden gut beherrschbare bzw. einstellbare und stabile Plasmakonzentrationen erreicht. Die Plasmakonzentration kann dabei leicht dosiert werden durch Variation beispielsweise der wirksamen Fläche des TTS bzw. dessen Größe. Weiterhin kann mittels der Applikation des TTS ein langsamer Anstieg (in Größenordnungen von Tagen und Wochen) der Plasmakonzentration des Wirkstoffes erreicht werden, mit dem Vorteil, dass anfängliche Nebenwirkungen vermieden werden. Zudem kann eine beispielsweise tägliche Applikation zu vorzugsweise frühen Tageszeiten (z.B. zwischen 06:00 und 15:00) eine unerwünschte Überstimulierung bei Nacht und das Risiko psychotischer Zustände zuverlässig vermeiden.

Nach Bedarf wird im Falle fortgeschrittener Erkrankungen die Behandlung durch Gaben von beispielsweise oralen oder parenteralen Zubereitungen mit dem gleichen dopaminergen Wirkstoff supplementiert. Die Tabletten weisen dabei vorzugsweise ein tmax von 15 bis 120 min., insbesondere 30 bis 60 min., und eine Halbwertszeit von 0,5 bis 4 Stunden, insbesondere 1 bis 2 Stunden, auf. tmax gibt die Zeitspanne an, die zwischen der oralen Gabe und der sich einstellenden maximalen Plasmakonzentration des Wirkstoffes der Tablette liegt. Die Halbwertszeit gibt die Zeitspanne an, in der sich die Plasmakonzentration, im abfallenden Zweig der Zeitfunktion, halbiert. Mittels der oralen Gabe und ihrer bedarfsgerechten rasch einsetzenden zusätzlichen Wirkung werden beispielsweise motorische Blockaden und Akinesie behoben, wann immer notwendig.

Wenn eine orale Gabe erst nach Beginn der kontinuierlichen Applikation des TTS durchgeführt wird, hat sich auch eine beachtliche Toleranz gegen dopaminerge Nebenwirkungen entwickelt und es ist dann nicht mehr erforderlich, langwierige (u.U. monatelange) "Titrationen" durchzuführen, wie dies zum Einstellen mit unterschiedlichen dopaminergen Wirkstoffen bei der Kombinationstherapie erforderlich ist. Dadurch wird eine besonders gute Verträglichkeit erreicht.

In Fällen, wo dies angezeigt ist, beispielsweise aufgrund der besonderen Schwere eines akuten Zustandes (z.B. morgendliche Akinesie oder Dystonie oder während "off" Perioden zu anderen Zeiten), kann auch eine parenterale Gabe (i.m., i.v., subkutan; Injektion oder Infusion) des gleichen Wirkstoffes, wie im TTS enthalten, erfolgen. Grundsätzlich gelten dabei die gleichen Vorteile, wie im Zusammenhang mit einer oralen Gabe bereits beschrieben. tmax liegt typischerweise unterhalb von 15 min., meist unterhalb von 5 min.

Im Falle von unerwarteten Nebenwirkungen ist aufgrund der kurzen Halbwertzeiten des Wirkstoffes ein längeres

Andauern der Nebenwirkungen zuverlässig vermeidbar. Durch lediglich Entfernen des TTS wird zusätzlich ein kurzfristiger Abfall der Plasmakonzentration an Wirkstoff erreicht. Dies ist insbesondere gegenüber den oral gegebenen und langzeitig wirkenden Wirkstoffen, wie Pergolid oder Cabergolin vorteilhaft, bei denen Nebenwirkungen in Folge einer Gabe bzw. Überdosierung über mehrere Tage andauern können.

Weiterhin werden mit der Erfindung vergleichsweise sehr hohe Gesamtaufnahmemengen des Wirkstoffes erreicht, da aufgrund der an sich komplexen Kinetiken und Wechselwirkungen, wenn unterschiedliche Stoffe kombiniert werden, aus Sicherheitsgründen zur Vermeidung von Nebenwirkungen stark unterdosiert wird. Mit der Erfindung wird also die klinische Wirksamkeit beachtlich erhöht. Dies, in Zusammenwirkung mit der besseren Verträglichkeit, erlaubt auch eine wesentlich längere Therapie mit dem einzelnen Wirkstoff und vermeidet gleichzeitig die Verwendung von Levodopa-Formulierungen. Dies ist insbesondere im Falle jüngerer Patienten mit hoher verbleibender Lebenserwartung von besonderer Bedeutung, da Levodopa (welches nach wie vor der "goldene Standard" dopaminerger Behandlung ist), bekanntermaßen LangzeitEffekte hervorruft, welche in schweren und unvorhersehbaren Dys- und Hyperkinesien resultieren, was Patienten letztendlich abhängig von fremder Hilfe und bettlägerig macht. Zudem zeigen Tierexperimente, dass selbst kurzzeitige Levodopa-Behandlung, auch in niedrigen Dosen im Zusammenhang mit den üblichen Kombinationstherapien, im Wege einer Art von "Anfachmechanismus" ein andauerndes "priming" bzw.

Sensibilisierung hervorruft mit der Folge von Langzeit-Komplikationen in den motorischen und mentalen Dopamin-Systemen. Bisher sind jedoch wegen der nebenwirkungsbedingten Unterdosierungen anderer dopaminerger Wirkstoffe die meisten Patienten bereits innerhalb der ersten Jahre der Erkrankung auf Levodopa-Gaben angewiesen und daher den abträglichen Langzeit-Nachteilen von Levodopa ausgeliefert.

Trotz der im Vergleich zu der Unterdosierung nach dem Stand der Technik vergleichsweise hohen Gesamtaufnahmemengen kann dennoch die Dosisbelastung niedrig gehalten werden (≤ 10 mg/Tag, insbesondere ≤ 5 mg/Tag), so dass die Behandlung relativ unabhängig von eventuellen Leber- oder Nierenfehlfunktionen durchgeführt werden kann. Zudem sind eventuelle Wechselwirkungen mit anderen Medikamenten als eher gering und vorhersehbar zu bewerten, da erfindungsgemäß nur noch ein einziger Wirkstoff zur Therapie involviert ist; Wechselwirkungen mit den sonst üblichen weiteren Parkinsonmitteln entfallen ganz.

Im Einzelnen kann die dopaminerg behandelbare Erkrankung eine Erkrankung aus der Gruppe bestehend aus "Morbus Parkinson, Parkinsonismus, Restless-Legs-Syndrom und Störungen des dopaminergen Systems" sein.

Der Dopaminagonist mit kurzer Halbwertszeit ist ein Ergolinderivat gemäß Formel I oder dessen physiologisch verträgliches Salz mit einer Säure ist,

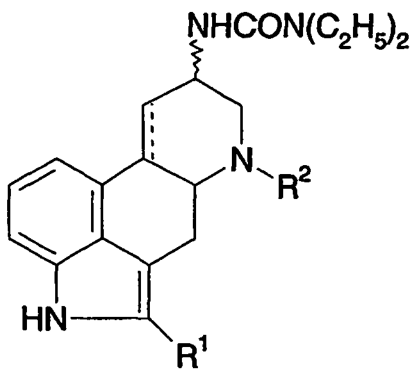

Formel I

worin --------- eine Einfachbindung oder eine Doppelbindung ist, worin R1 ein H-Atom oder ein Halogenatom, insbesondere ein Bromatom ist, und worin R2 C1-4-Alkyl, insbesondere Methyl, ist.

Als Ergolinderivate kommen insbesondere in Frage: Lisurid, Bromlisurid (3-(2-Brom-9,10-didehydro- 6-methyl-8a-ergolinyl)-1,1-diethylharnstoff), Tergurid (3-(6-methyl-8a-ergolinyl)-1,1-diethyl-harnstoff) und Protergurid (3-(6-propyl-8a-ergo- linyl)-1,1-diethylharnstoff). Bevorzugt ist es allerdings, wenn das Ergolin-Derivat Lisurid (3-(9,10-didehydro-6-methyl-8a-ergolinyl)-1,1-diethyl-harnstoff) oder dessen physiologisch verträgliches Salz mit einer Säure ist. In Frage kommende Salze der Wirkstoffe sind beispielsweise Sulfate, Phosphate, Maleate, Citrate und Succinate sowie insbesondere Hydrogenmaleat.

Je nach Freisetzungskinetik kann das TTS in diversen Zeitabständen appliziert werden. Wesentlich ist lediglich, dass die Plasmakonzentration bei der kontinuierlichen Anwendung des TTS nicht in störendem Maße schwanken. Bevorzugt ist es, wenn das TTS täglich appliziert wird.

Die zur oralen oder parenteralen Gabe hergerichtete Zubereitung wird vorzugsweise unmittelbar bei akutem Auftreten von dopaminerg bedingten Fehlfunktionen verabreicht. Es ist aber auch eine präventive Verabreichung möglich im Falle von vorhersehbaren Fehlfunktionen.

Der Begriff des TTS umfasst insbesondere perkutan wirkende, aber auch transmucosal wirkende Systeme. Ein TTS ist typischerweise von flächiger Struktur und wird beispielsweise auf der Haut flächig zum Anliegen gebracht. Ein TTS umfasst meist eine einen Wirkstoff (ggf. in Salzform) enthaltende Matrix und/oder ein Wirkstoffreservoir und hautseitig des Wirkstoffreservoirs eine wirkstoffpermeable Diffusionsbarriere. Die Befestigung auf der Haut kann durch ein ggf. zusätzliches hautseitiges (und für den Wirkstoff permeables) Adhäsiv erfolgen. Ebenso kann die Matrix und/oder die Diffusionsbarriere selbst mit adhäsiven Eigenschaften ausgestattet sein. Schließlich kann ein nicht-adhäsives TTS mittels weiterer Hilfsmittel, beispielsweise Klebebänder oder Bandagen, auf der Haut zum Anliegen gebracht werden. Als Matrix ist ein Stoff bezeichnet, in welchem der Wirkstoff immobilisiert ist. Demgegenüber ist der Wirkstoff in einem Wirkstoffreservoir nicht notwendigerweise immobilisiert, weswegen das Wirkstoffreservoir ummantelt sein muss. Der hautseitige Teil des Mantels wird dabei von der Diffusionsbarriere gebildet. Es versteht sich, dass der weitere Teil des Mantels möglichst inpermeabel, auch bezüglich Diffusionspfade, für den Wirkstoff sein sollte. Der Begriff immobilisiert meint in diesen Zusammenhängen, dass kein unkontrollierter Wirkstoff-Fluss möglich ist. Insbesondere Diffusion eines Wirkstoffes in einer Matrix und/oder durch eine Diffusionsbarriere ist jedoch nicht nur möglich, sondern gezielt eingerichtet. Die Diffusionskoeffizienten bestimmen dabei letztendlich den Flux des Wirkstoffes aus dem TTS in die Haut eines Patienten. Die an die Haut eines Patienten abgegebene Dosis ist daher eine in erster Näherung lineare Funktion der wirksamen Fläche des TTS. Die wirksame Fläche ist die Kontaktfläche von für Wirkstoffe diffusionsoffenen Bereichen des TTS.

Ein TTS des vorstehend genannten Aufbaus mit Lisurid als Wirkstoff sowie dessen Verwendung zur Behandlung der Parkinson'schen Krankheit ist grundsätzlich bekannt aus der Literaturstelle WO 92/20339. Hierin ist insbesondere der Effekt von Propylenglycol-Laurinsäure auf den Flux beschrieben, wodurch eine beachtliche Fluxerhöhung erreicht wird. Ein Lisurid enthaltendes TTS ist weiterhin bekannt aus der Literaturstelle WO 91/00746. Selbstverständlich kann der Wirkstoff in einem transdermalen Pflaster gemäß den aus dem Stand der Technik bekannten pharmazeutischen Verfahren formuliert sein.

Die gleichzeitige orale und transdermale Gabe zur Verbesserung des Freigabeprofils und Verringerung der Nebenwirkungen ist zum Beispiel aus US-4920989 bekannt. Es wird darin eine Methode zur Entwöhnung von Rauchern beschrieben, wobei ein transdermales nikotinhaltiges Pflaster zusammen mit ein nikotinhaltiges Mundspray verwendet werden.

Im Einzelnen ist es bevorzugt, wenn das TTS eine Arzneimittelschicht aufweist, welche zumindest eine einen Wirkstoff enthaltende Matrix und/oder ein Wirkstoffreservoir und hautseitig des Wirkstoffreservoirs eine wirkstoffpermeable Diffusionsbarriere sowie als Wirkstoff ein Ergolin-Derivat gemäß Formel I oder dessen Salz enthält.

Die Matrix und/oder die Diffusionsbarriere kann ausgewählt sein mit der Maßgabe, dass der transdermale Fluss F durch Humanhaut, gemessen gemäß Beispiel 1, im Bereich von 0,1 bis 5,0 $\mu g/cm^2/h$, insbesondere 0,1 bis 4,0 $\mu g/cm^2/h$, liegt.

Vorzugsweise ist ein TTS-Set als Bestandteil des Mittels eingerichtet, wobei das Set eine Mehrzahl von TTS-Elementen enthält und wobei die Elemente zur Abgabe von unterschiedlichen Dosen eingerichtet sind. Die TTS-Elemente können voneinander separiert und mit einer kontinuierlich ansteigenden Folge für F im Bereich von 0,1 bis 5 $\mu g/cm^2/h$ eingerichtet sein. Es sind aber auch mehrere TTS mit gleichem F-Wert innerhalb einer Untergruppe möglich, wobei der F-Wert verschiedener Untergruppen eine kontinuierlich ansteigende Folge bildet und weitere Untergruppen konstante F-Werte mit maximalem F-Wert aus vorstehender Folge aufweisen. Vorzugsweise werden F und wirksame Fläche des TTS so ausgewählt, dass sich ab dem zweiten Tag der Applikation eine (subsequent in Stufen ansteigende) Dosis im Bereich von 10 $\mu g$ bis 2 mg Wirkstoff (beispielsweise Lisurid) über den Tag bzw. 24 Stunden einstellt. Die TTS-Elemente können auch in kontinuierlicher Folge mit unterschiedlicher wirksamer Fläche ausgestattet sein. Unterteilungen in Untergruppen im vorstehenden Sinne sind auch hier möglich. Erfindungsgemäß geeignet sind auch andere aus dem Stand der Technik bekannte gängige transdermale Applikationsformen.

Die zur oralen Gabe hergerichtete Zubereitung kann in Form einer Tablette, als Pulver, in einer Kapsel oder als Lösung erfolgen und ist je nach Applikationsart gemäß den aus dem Stand der Technik bekannten Verfahren formuliert und enthält in Tablettenform vorzugsweise 25 bis 1000 $\mu g$ des dopaminergen Wirkstoffes (je Tablette), beispielsweise für Lisurid von 0,075 mg bis 5,0 mg pro Tag.

Die zur parenteralen Gabe hergerichtete Zubereitung in Form einer Injektions- oder Infusionslösung ist gemäß den bekannten Verfahren formuliert und enthält vorzugsweise 25 bis 2000 $\mu g$ des dopaminergen Wirkstoffes (je ml Lösung). Für eine rasche zusätzliche Wirkung bei bereits bestehender Toleranz beträgt die parenterale Dosis beispielsweise für Lisurid bis zu 5,0 mg bei Dauerinfusion über 24 oder 16 Stunden und von 25 bis 200 $\mu g$ im Bolus einer einzelnen Applikation.

Das TTS kann im Detail wie folgt ausgebildet sein. Auf der hautabgewandten Seite der Matrix und/oder des Wirkstoffreservoirs kann eine Deckschicht angeordnet sein. Diese kann beispielsweise mit Folien aus Polyethylen oder

Polyester gebildet sein. Die Dicke beträgt typischerweise 10 bis 100 µm. Es ist möglich zur Erzielung eines ausreichenden Lichtschutzes, die Deckschicht zu pigmentieren, zu lackieren und/oder zu metallisieren. Als Metallisierung ist das Aufbringen einer sehr dünnen Schicht (typischerweise weniger als 1 µm, meist im 10-100 nm Bereich) eines Metalls, beispielsweise Aluminium, auf die Deckschicht bezeichnet. Pigmente können alle im Rahmen der Überzugsmittel gebräuchlichen Pigmente, auch Effektpigmente, sein, sofern sie physiologisch unbedenklich sind. Auf der Applikationsseite kann ein abziehbarer Liner vorgesehen sein, beispielsweise eine silikonisierte oder fluorpolymerbeschichtete polymere Schutzfolie.

Die Matrix und/oder Diffusionsbarriere kann einen Stoff, ausgewählt aus "Polyacrylat, Polyurethan, Celluloseether, Silikon, Polyvinylverbindungen, Polyisobutylenverbindungen, Silikat und Mischungen dieser Stoffe sowie Copolymere dieser Polymerverbindungen", vorzugsweise Polyacrylat, als Hauptmatrixkomponente aufweisen. Eine Hauptmatrixkomponente bildet zumindest 50 Gew.-%, beispielsweise zumindest 80-90 Gew.-% der Matrix (der Begriff der Matrix bezieht sich dabei auf die fertige Schicht, i.e. Hauptmatrixkomponente(n) mit Hilfsstoff(en) und Wirkstoff(en)). Eine Einstellung des gewünschten Flux erfolgt einerseits durch Auswahl des Stoffes in Abhängigkeit des Diffusionskoeffizienten des Wirkstoffes darin und andererseits und ggf. in Abstimmung hiermit durch Wahl der Schichtdicke der Matrix in Richtung orthogonal zur Hautoberfläche. Der Dickenbereich einer Matrix liegt typischerweise im Bereich von 10 µm bis 500 µm.

Ein besonders bevorzugter Polyacrylatkleber als Hauptmatrixkomponente ist käuflich unter der Bezeichnung GELVA® multipolymer solution 7881, erhältlich von der Firma Monsanto Deutschland GmbH, Düsseldorf. Dabei wird ausdrücklich Bezug genommen auf das unter dieser Bezeichnung vertriebe Produkt gemäß Datenblatt in der Fassung vom 23.04.1996. Ebenfalls gut verwendbar ist Eudragit® E100, erhältlich von der Firma Röhm, Deutschland.

Mit den vorstehenden Polyacrylatklebern wird eine besonders vorteilhafte nichttriviale Eigenschaftskombination erhalten, nämlich optimaler Flux, gute Haftfähigkeit, gute Hautverträglichkeit und gute Haltbarkeit.

Die Diffusionsbarriere kann alternativ ein Polymer ausgewählt aus "Celluloseester, Celluloseether, Silikon, Polyolefin und Mischungen sowie Copolymere dieser Stoffe" als Hauptbarrierenkomponente aufweisen. Zum Begriff der Hauptbarrierenkomponente gilt das Vorstehende zur Hauptmatrixkomponente analog. Die Diffusionsbarriere kann als Folie mit einer Dicke von 10 µm bis 300 µm ausgebildet sein, wobei die Dicke der Schicht (in Verbindung mit dem Diffusionskoeffizienten des Wirkstoffes in dem Polymer) nach Maßgabe des gewünschten Flux eingestellt wird.

In der Matrix und/oder dem Wirkstoffreservoir und/oder der Diffusionbarriere können für TTS übliche Hilfsstoffe enthalten sein. Bevorzugterweise wird als Hilfsstoff ein penetrationsverstärkendes Mittel eingesetzt, welches vorzugsweise ausgewählt ist aus "C1-C8 aliphatische, cycloaliphatische und aromatische Alkohole, gesättigte und ungesättigte C8-18-Fettalkohole, gesättigte und ungesättigte C8-18 Fettsäuren, Kohlenwasserstoffe und Kohlenwasserstoffmischungen, Fettsäureester aus C3-19-Fettsäuren und C1-6-Alkylmonoolen, Dicarbonsäuredieester aus C4-8-Dicarbonsäuren und C1-6-Alkyl-monoolen, und Mischungen dieser Stoffe. Penetrationsverstärkende Mittel verbessern den Flux des Wirkstoffes durch die Haut, auf welche das TTS aufgebracht ist. Beispiele aus den vorgenannten Stoffen sind: 1,2-Propandiol, Menthol, Dexpanthenol, Benzylalkohol, Laurylalkohol, Isocetylalkohol, Cetylalkohol, Mineralöl, Laurinsäure, Isopalmitinsäure, Isostearinsäure, Ölsäure; Methylester, Ethylester, 2-Hydroxyethylester, Glycerolester, Propylester, Isopropylester, Butylester, sec.-Butylester oder Isobutylester der Laurinsäure, Myristinsäure, Stearinsäure oder Palmitinsäure. Bevorzugt ist der Einsatz von Dimethylisosorbid, Isopropylmyristat und Laurylalkohol, höchstbevorzugt von Laurylalkohol. Als weitere Hilfsstoffe kommen beispielsweise Kristallisationsinhibitoren in Frage. Als Kristallisationsinhibitoren sind hochdisperses Siliziumdioxid oder makromolekulare Stoffe wie Polyvinylpyrrolidone, Polyvinylalkohole, Dextrine, Dextrane,Sterine, Gallensäuren und insbesondere Polyvinylpyrrolidon-Vinylacetat-Copolymere geeignet wie Kollidon® VA 64.

Es versteht sich, dass jedenfalls das penetrationsverstärkende Mittel ebenfalls ausreichend durch die Matrix bzw. die Diffusionsbarriere diffundieren muss. Im Falle des Einsatzes einer Matrix sowie des Hilfsstoffes Laurylalkohol bildet der Laurylalkohol vorzugsweise 10 bis 30 Gew.-%, höchstvorzugsweise 15 bis 20 Gew.-%, der Matrix.

Die Hilfsstoffe können grundsätzlich 0 bis 50 Gew.-% der Matrix bilden. Der Wirkstoff kann 0,5 bis 20 Gew.-%, vorzugsweise 1 bis 10 Gew.-%, der Matrix bilden. Die Summe der Anteile an Matrixhauptkomponente, Hilfsstoffen und Wirkstoffen bildet dabei stets 100 Gew.-%.

Die Dosis des Wirkstoffes in einem das TTS tragenden menschlichen Körper hängt neben den vorstehenden diffusionsbezogenen Eigenschaften des TTS auch von dessen wirksamer Fläche mit der Haut ab. Wirksame Fläche meint hierbei die Fläche, mit welcher die Matrix oder die Diffusionsbarriere an der Haut anzuliegen kommt. Vorzugsweise erfolgt die Variation nach Maßgabe der gewünschten Dosis in einem Bereich von 1 bis 100 $cm^2$. Im Rahmen der Erfindung lassen sich dabei, bei abgestimmten Flux für eine vorgegebene Indikation, leicht patientenindividuelle Dosisvariationen von einem Arzt einrichten, nämlich durch Wahl einer geeigneten Größe. Somit kann die Behandlung beispielsweise auf unterschiedliche Körpergewichte, Altersgruppen etc. unschwer abgestellt werden. Insbesondere ist es möglich, ein TTS, welches eine (eher große) Standardfläche aufweist, mit Unterteilungsmarkierungen für Teildosen auszustatten, so dass ein Anwender lediglich einen einer bestimmten Dosis entsprechenden Teilabschnitt abtrennen und anwenden kann. Entsprechende Aufdrucke lassen sich unschwer auf der Deckschicht anbringen.

Zur Monotherapie dopaminerger Erkrankungen kann eine transdermale und eine orale bzw. parenterale Applikationsform eines Wirkstoffes in Form einer Kombinationspackung (Kit) angeboten werden.

Die Erfindung betrifft auch die Kombination eines transdermalen therapeutischen Systems und einer oralen und/oder parenteralen Zubereitung enthaltend als Wirkstoff den gleichen Dopaminagonisten mit kurzer Halbwertszeit zur Herstellung eines Arzneimittels zur Behandlung dopaminerger Erkrankungen.

Beispiel 1: Flux-Messung

Zur Flux-Messung wird eine FRANZ Durchfluss Diffusionszelle verwendet. Die Messfläche beträgt 2 $cm^2$. Als Hautprobe werden 4 $cm^2$ ventrale und dorsale Haut einer männlichen haarlosen Maus (MF1 hr/hr Ola/Hsd, erhältlich von Harlan Olac, UK) verwendet, wobei subkutanes Fettgewebe sorgfältig entfernt wird. Auf die eingesetzte Haut ist ein 2 $cm^2$ TTS appliziert. Gegenüberliegend ist das Akzeptormedium angeordnet. Es ist verdünntes HHBSS (Hepes Hanks Balanced Salt Solution) enthaltend 5,96 g/l Hepes, 0,35 g/l $NaHCO_3$ und 0,1 ml/l 10x HBSS (erhältlich von Gibco, Eggenstein, DE). Weiterhin sind 1000 I.E./ml Penicillin (Benzylpenicillin Kaliumsalt, erhältlich von Fluka, Neu-Ulm, DE).

Die Messung erfolgt im einzelnen wie folgt. Das zu messende TTS wird zunächst auf die Haut appliziert. Sofort danach wird die Haut in die Diffusionszelle montiert. Das Akzeptormedium wird in Intervallen von 2h zwischen t=0 und t=6 h und von 8 h zwischen t=6 h und t=54h beprobt. Pro Stunde werden 1ml Akzeptormedium durch die Diffusionszelle mittels einer peristaltischen Pumpe gepumpt. Die Temperatur des Akzeptormediums wird mittels eines zirkulierenden Wasserbades kontrolliert und hält die Oberfläche der Haut auf einer Temperatur von 31 °C mit 1 °C Genauigkeit.

Die Wirkstoffkonzentration in dem Akzeptormedium wird gemäß folgender Details mittels eines Radioimmunoassays bestimmt.

Kalibrierungskurven: Diese werden unter Verwendung von zwei unterschiedlichen Methanollösungen von nicht radioaktivem Lisuridhydrogenmaleatsalz, enthaltend je 1 mg/ml, konstruiert. Diese Lösungen werden unterschiedlich mit BSA-Puffer (0,041 M $Na_2HPO_2$*$2H_2O$, 0,026 M $KH_2PO_4$, 0,154 M NaCl, 0,015 M $NaN_3$, 0,1% (w/v) BSA, pH 7, supplementiert mit 0,05% (w/v) Ascorbinsäure) verdünnt, um Lisurid-free-base-Konzentrationen im Bereich von 1000 - 3,9 pg/0,1ml zu erhalten. Zusätzlich wird eine wirkstofffreie Probe (0pg) eingesetzt. Die Kalibrierungsproben werden dreifach analysiert. Die Lisurid-Konzentrationen werden mittels der pharmacokinetic RIO PC Software, 2.5, berechnet (andere übliche Software ist ebenfalls einsetzbar).

Probenpräparation: Vor der Analyse wird das Akzeptormedium mit BSA-Puffer verdünnt zwecks Einstellung von Konzentrationen im auswertbaren Bereich der Kalibrierungskurve. 100 μl verdünnte Probe werden direkt der radioimmunologischen Analyse unterzogen.

Antiserum: Das Antiserum (Kaninchen) ist erhältlich durch Immunisierung mit dem Immunogen Lisurid-1-succinyl-BSA. Die Verdünnung des Antiserums im Assay ist 1:12500.

Tracer: $^3$H-Lisuridhydrogenmaleat mit einer spezifischen Aktivität von 4,3 GBq/mg wird verwendet.

Inkubation: zu 0,7 ml BSA-Puffer werden 0,1 ml BSA-Puffer mit Wirkstoff, 0,1 ml Tracerlösung (ca. 5000 cpm/0,1 ml BSA-Puffer) und 0,1 ml verdünntes Antiserum (1:12500) gegeben und es wird für 18 h bei 4°C inkubiert.

Separierung: antikörpergebundenes Lisurid wird von freiem durch Zugabe von 0,2 ml Holzkohlesuspension (1,25%(w/v) und 0,125% (w/v) Dextran in BSA-Puffer) und Inkubation für 30 min. bei 0 °C getrennt. Die Holzkohle wird durch Zentrifugation bei 3000 g für 15 min. sedimentiert. Der Überstand (enthaltend antikörpergebundenen Wirkstoff) wird dekantiert und der radiometrischen Analyse zugeführt.

Radiometrische Analyse: Zum Überstand werden 4ml des Szintillations Cocktails Atomlight (NEN) gegeben. Die Zählung erfolgt mit einem WALLAC 1409 oder 1410 β-Szintillationszähler ohne quench control.

Auswertung: Der perkutane Haut Flux wird wie folgt berechnet:

$$F = (C * R) / (A * T),$$

wobei F den percutanen Flux [ng/$cm^2$/h], C die Wirkstoffkonzentration im Akzeptormedium [ng/ml], R den Akzeptormediumsfluss [1ml/h], A die Messfläche [2$cm^2$] und T das Beprobungszeitintervall [h] sind.

Maximaler transdermaler Wirkstoffflux wird direkt von den Daten genommen. Mittlere perkutane Fluxwerte werden während Tag 1 und Tag 2 des Experiments bestimmt, basierend auf der kumulativ absorbierten Dosis in dem Zeitintervall t=0-22 und t=22-54.

Beispiel 2: Herstellung eines TTS A

15 mg Kollidon VA 64 (Kristallisationsinhibitor) werden in 15 mg Isopropanol gelöst. Dann werden 5 mg Lisurid

eingestreut. 80mg Polyacrylatkleber (Gelva 7881) werden in einem Becherglas vorgelegt und die vorstehende Suspension, unter Nachspülen mit 30 mg Isopropanol, hinzugegeben. Nach gründlicher Durchmischung wird das erhaltene kristallfreie Wetmix mit 500 µm Rakel auf einem silikonisierten Liner ausgezogen. Dann wird bei 60 °C für 20 min. getrocknet und schließlich eine Deckschicht auflaminiert.

Messungen des Flux gemäß Beispiel 1 ergeben für F einen Tag 1 Wert von 0,43, einen Tag 2 Wert von 0,44 und einen maximalen F von 0,85 (jeweils in µg/cm$^2$/h).

Beispiel 3: Herstellung eines TTS B

12,5 mg Dimethylisosorbid werden mit 2 mg Lisurid in 15 mg Isopropanol suspendiert. 80 mg Polyacrylatkleber (Gelva 7881) werden in einem Becherglas vorgelegt und die vorstehende Suspension, unter Nachspülen mit 30 mg Isopropanol, hinzugegeben. Nach gründlicher Durchmischung wird das erhaltene kristallfreie Wetmix mit 500 µm Rakel auf einem silikonisierten Liner ausgezogen. Dann wird bei 60 °C für 20 min. getrocknet und schließlich eine Deckschicht auflaminiert.

Messungen des Flux gemäß Beispiel 1 ergeben für F einen Tag 1 Wert von 0,23, einen Tag 2 Wert von 0,28 und einen maximalen F von 0,50 (jeweils in µg/cm$^2$/h).

Beispiel 4: Herstellung eines TTS C

27,2 mg Kollidon VA 64 (Kristallisationsinhibitor) und 16,3 mg Laurylalkohol werden bei 60 °C gelöst. Dann werden 2 mg Lisurid in dieser Lösung bei 60 °C gelöst. 39,38 mg Eudragit E100, 13,41 mg Citroflex 4A und 1,71 mg Bernsteinsäure werden bei 150-200 °C geschmolzen. Nach Abkühlung auf 80 °C wird die Lisuridlösung unter Rühren hinzugegeben. Bei 80 °C wird mit 500 µm Rakel auf einem silikonisierten Liner ausgezogen. Dann wird auf 20 °C abgekühlt und schließlich ggf. eine Deckschicht auflaminiert.

Messungen des Flux gemäß Beispiel 1 ergeben für F einen Tag 1 Wert von 0,90, einen Tag 2 Wert von 1,76 und einen maximalen F von 2,53 (jeweils in µg/cm$^2$/h).

Beispiel 5: Herstellung einer Zubereitung zur oralen Gabe

Eine Tablettenbasis-Zusammensetzung enthaltend Lactose, microkristalline Cellulose, Maisstärke, Crosscarmellose und Magnesiumstearat in üblicher Mengenzusammensetzung wird mit 2000 µg Lisurid je g Tablettenbasiszusammensetzung vermischt und zu Tabletten verpresst, welche jeweils 200 µg Lisurid enthalten.

Beispiel 6: Herstellung einer Zubereitung zur parenteralen Gabe.

Eine Injektionsbasislösung enthaltend Lactose, NaCl und aqua p.i. in üblicher Mengenzusammensetzung wird mit 50 µg Lisurid je g Injektionsbasislösung vermischt und in Braunglasampullen enthaltend 50 µg Lisurid je ml Lösung abgefüllt und vorzugsweise lyophilisiert.

Beispiel 7: Herstellung eines erfindungsgemäßen Mittels

Eine Mehrzahl von TTS, aufgeteilt in vier Gruppen, gemäß Beispiel 2 wird zusammengestellt. Dabei weisen die TTS jeder Gruppe jeweils einen Fluss F an Lisurid durch Human Haut von 0,25 µg/cm$^2$/h, 0,5 µg/cm$^2$/h, 0,75µ g/cm$^2$/h und 1,0 µg/cm$^2$/h auf. In den drei Gruppen mit niedrigem F sind zumindest jeweils 7 TTS vorgesehen. In der Gruppe mit höchstem F sind 28 oder mehr TTS vorgesehen. Den zusammengestellten TTS werden eine Mehrzahl von Tabletten aus Beispiel 5 und/oder eine Mehrzahl von Ampullen aus Beispiel 6 beigepackt. Der so erhaltenen Zusammenstellung wird ein Beipackzettel mit u.a. den Hinweisen auf den erfindungsgemäßen Behandlungsplan beigelegt.

Beispiel 8: Behandlung eines an der Parkinson'schen Erkrankung erkrankten Patienten.

Einem Parkinson-Patienten wird über einen Zeitraum von 28 Tagen an jedem Tag morgens ein TTS aus Beispiel 7 appliziert. Dabei ist die Fläche des TTS über 7 aufeinanderfolgende Tage unverändert. Die in subsequenten 7-Tage Folgen applizierten TTS weisen eine zunehmende Fläche auf, so dass sich eine in vier Stufen erhöhte mittlere Plasmakonzentration (gemittelt über einen Tag) an Lisurid ergibt. Der Fluss F an Lisurid der in den vier Stufen eingesetzten TTS ist dabei 0,25 µg/cm$^2$/h, 0,5 µg/cm$^2$/h, 0,75 µg/cm$^2$/h und 1,0 µg/cm$^2$/h. Anschließend an Tag 28 wird mit der täglichen, kontinuierlichen Applikation eines TTS mit einem F von 1,0 µg/cm$^2$/h fortgefahren, i.e. ohne weitere Erhöhung der Dosis. Es versteht sich, dass mit jeder Applikation eines neuen TTS das alte entfernt wird.

Ab Tag 28 wird bei akuten Zuständen, beispielsweise schweren Dystonien, oral eine Tablette aus Beispiel 7

gegeben oder der Inhalt einer Ampulle aus Beispiel 7 i.m. injiziert. Stattdessen oder zusätzlich kann morgens eine Tablette aus Beispiel 7 oder der Inhalt einer Ampulle aus Beispiel 7 aus präventiven Gründen gegeben werden.

Der Patient zeigt zu keiner Zeit der Behandlung beachtliche Nebenwirkungen. Insbesondere die oralen oder parenteralen Gaben anlässlich akuter Zustände werden gut vertragen und auch daran anschließend wird keine nennenswerte Störung des normalen REM-Schlafes beobachtet.

Treten wider Erwarten dennoch störende Nebenwirkungen auf, so können diese durch ersatzlose Entfernung des TTS und folglich kurzfristiger Reduktion der Plasmakonzentration an Lisurid wirkungsvoll gedämpft werden.

## Patentansprüche

1. Verwendung eines Ergolin-Derivats gemäss Formel I oder dessen physiologisch verträglichem Salz,

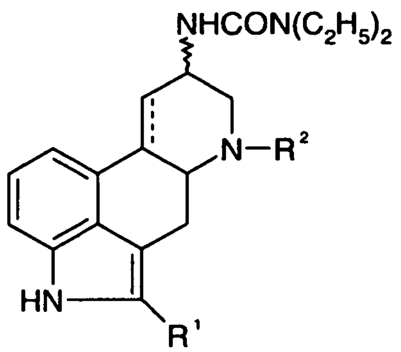

**Formel I**

worin <u>---------</u> eine Einfachbindung oder eine Doppelbindung ist, worin R1 ein H-Atom oder ein Halogenatom, insbesondere ein Bromatom ist, und worin R2 C1-4-Alkyl ist, zur Herstellung eines Mittels zur Behandlung von Erkrankungen aus der Gruppe bestehend aus Morbus Parkinson, Parkinsonismus, Restless-Legs-Syndrom und Störungen des dopaminergen Systems, **dadurch gekennzeichnet, dass**
das Mittel in mindestens zwei räumlich getrennten Formen vorliegt, wovon eine das Ergolin-Derivat in einem transdermalen therapeutischen System (TTS) enthält, und eine oder mehrere weitere Formen dasselbe Ergolin-Derivat in einer zur oralen und/oder parenteralen Gabe hergerichteten Zubereitung enthalten,
und dass das TTS für eine kontinuierliche Applikation vorgesehen ist und die zur oralen und/oder parenteralen Gabe hergerichtete Zubereitung für eine diskontinuierliche Gabe innerhalb der Applikationsdauer des TTS vorgesehen ist.

2. Verwendung nach Anspruch 1, wobei das Ergolin-DerivatLisurid oder eines seiner pharmazeutisch verträglichen Salze ist.

3. Verwendung nach einem der Ansprüche 1 bis 2, wobei das Ergolin-Derivateine Halbwertszeit von 0,5 bis 4 Stunden, insbesondere von 1 bis 2 Stunden hat.

4. Verwendung nach einem der Ansprüche 1 bis 3, wobei das TTS eine Arzneimittelschicht aufweist, welche zumindest eine einen Wirkstoff enthaltende Matrix und/oder ein Wirkstoffreservoir und hautseitig des Wirkstoffreservoirs eine wirkstoffpermeable Diffusionsbarriere aufweist.

5. Verwendung nach Anspruch 4, wobei die Matrix und/oder die Diffusionsbarriere ausgewählt ist mit der Maßgabe, dass der transdermale Fluss F durch Humanhaut im Bereich von 0,1 bis 5,0 $\mu g/cm^2/h$ liegt.

6. Verwendung nach einem der Ansprüche 4 bis 5, wobei auf der hautabgewandten Seite der Matrix und/oder des Wirkstoffreservoirs eine Deckschicht angeordnet ist.

7. Verwendung nach einem der Ansprüche 4 bis 6, wobei die Matrix und/oder Diffusionsbarriere einen Stoff, ausgewählt aus Polyacrylat, Polyurethan, Celluloseether, Silikon,Polyvinylverbindungen, Polyisobutylenverbindungen, Silikat und Mischungen dieser Stoffe sowie Copolymere dieser Polymerverbindungen, vorzugsweise Polyacrylat, als Hauptmatrixkomponente aufweist.

**8.** Verwendung nach einem der Ansprüche 4 bis 7, wobei die Diffusionsbarriere ein synthetisches Polymer ausgewählt aus Celluloseester, Celluloseether, Silikon, Polyolefin und Mischungen sowie Copolymere dieser Stoffe als Hauptbarrierenkomponente aufweist.

**9.** Verwendung nach einem der Ansprüche 4 bis 8, wobei die Matrix und/oder das Wirkstoffreservoir und/oder die Diffusionsbarriere ein penetrationsverstärkendes Mittel enthält, welches ausgewählt ist aus C1-C8 aliphatische, cycloaliphatische und aromatische Alkohole, gesättigte und ungesättigte C8-18 Fettalkohole, gesättigte und ungesättigte C8-18 Fettsäuren, Kohlenwasserstoffe und Kohlenwasserstoffmischungen, Fettsäureester aus C3-19-Fettsäuren und C1-6-Alkylmonoolen, Dicarbonsäurediester aus C4-8-Dicarbonsäuren und C1-6-Alkylmonoolen, und Mischungen dieser Stoffe.

**10.** Verwendung nach einem der Ansprüche 1 bis 9, wobei ein TTS Set eingerichtet ist, wobei das Set eine Mehrzahl von TTS-Elementen enthält und wobei die Elemente zur Abgabe von unterschiedlichen Dosen eingerichtet sind.

**11.** Verwendung nach einem der Ansprüche 1 bis 9, wobei die zur oralen Gabe hergerichtete Zubereitung in Tablettenform 25 bis 500 μg des Ergolin-Derivats je Tablette enthält.

**12.** Verwendung nach einem der Ansprüche 1 bis 9, wobei die zur parenteralen Gabe hergerichtete Zubereitung in Form einer Injektions- oder Infusionslösung 25 bis 2000 μg des Ergolin-Derivatsg je ml Lösung enthält.

**Claims**

**1.** Use of an ergoline derivative according to formula I or a physiologically compatible salt thereof,

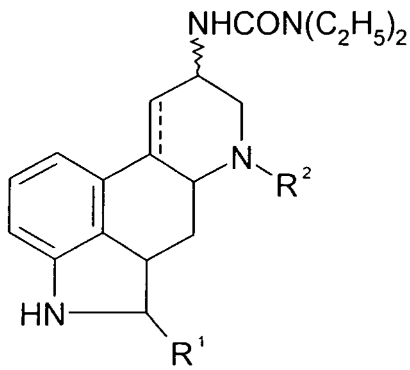

Formula I

where --------- is a single or double bond wherein R1 is an H atom or a halogen atom, particularly a bromine atom, and wherein R2 is C1-4 alkyl
for the preparation of an agent for treatment of diseases selected from the group consisting of Parkinson's disease, parkinsonism, restless legs syndrome, and disturbances of the dopaminergic system, **characterized in that** the agent has at least two spatially discrete compositions, of which one contains the ergoline derivative in a transdermal therapeutic system (TTS) and another one or more further forms contain that same ergoline derivative in a preparation for oral and/or parenteral application
and **in that** the TTS is intended for a continuous application and the preparation for oral or parenteral application is intended for a discontinuous application during the application period of the TTS.

**2.** The use according to claim 1 wherein the ergoline derivative is lisuride or a pharmaceutically compatible salt thereof.

**3.** The use according to any one of claims 1 to 2 wherein the ergoline derivative has a half-life of 0.5 to 4 hours,

preferably 1 to 2 hours.

4. The use according to any one of claims 1 to 3 wherein the TTS has a pharmaceutical layer, which comprises at least one matrix containing an active ingredient and/or an active ingredient reservoir, and a diffusion barrier on the skin side of said active ingredient reservoir that is permeable to said active ingredient.

5. The use according to claim 4 wherein the matrix and/or diffusion barrier are selected so that the transdermal flux F through human skin is in the range from 0.1 to 5.0 μg/$cm^2$/h.

6. The use according to any one of claims 4 to 5 wherein a covering layer is provided on the side of the matrix and/or active ingredient reservoir that faces away from the skin.

7. The use according to any one of claims 4 to 6 wherein the matrix and/or diffusion barrier has as their main matrix component a substance selected from polyacrylate, polyurethane, cellulose ether, silicone, polyvinyl compounds, polyisobutylene compounds, silicate and mixtures of these substances as well as copolymers of these polymeric compounds, preferably polyacrylate.

8. The use according to any one of claims 4 to 7 wherein the diffusion barrier has as its main barrier component a synthetic polymer selected from cellulose ester, cellulose ether, silicone, polyolefin and mixtures as well as copolymers of these substances.

9. The use according to any one of claims 4 to 8 wherein the matrix and/or the active ingredient reservoir and/or the diffusion barrier contain a penetration-enhancing agent that is selected from C1-C8 aliphatic, cycloaliphatic and aromatic alcohols, saturated and unsaturated C8-18 fatty alcohols, saturated and unsaturated C8-18 fatty acids, hydrocarbons and hydrocarbon mixtures, fatty acid esters from C3-19 fatty acids and C1-6 alkyl monools, dicarboxylic acid diesters from C4-8 dicarboxylic acids and C1-6 alkyl monools, and mixtures of these substances.

10. The use according to any one of claims 1 to 9 wherein a TTS set is provided containing a multitude of TTS elements and wherein the elements are designed for the release of different doses.

11. The use according to any one of claims 1 to 9 wherein the preparation for oral administration in tablet form contains 25 to 500 μg of the ergoline derivative per tablet.

12. The use according to any one of claims 1 to 9 wherein the preparation for parenteral administration in form of an injection or infusion solution contains 25 to 2000 μg of the ergoline derivative per ml solution.

**Revendications**

1. Utilisation d'un dérivé d'ergoline selon la formule I ou son sel physiologiquement acceptable,

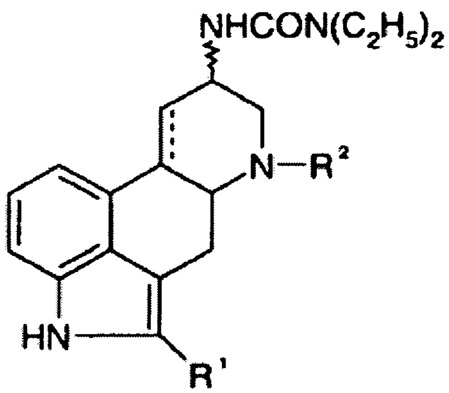

Formule I

dans laquelle --------- est une liaison simple ou une liaison double, R1 étant un atome d'hydrogène ou un atome d'halogène, en particulier, un atome de brome et R2 étant un groupe alkyle en C1 à C4, pour produire un agent de traitement de maladies comprises dans le groupe constitué de la maladie de Parkinson, du parkinsonisme, du syndrome des jambes sans repos et des troubles du système dopaminergique, **caractérisée en ce que** l'agent se présente dans au moins deux formes séparées spatialement, dont l'une contient le dérivé d'ergoline dans un système thérapeutique transdermique (TTS) et une ou plusieurs autres formes contiennent ce même dérivé d'ergoline dans une préparation adaptée pour l'administration orale et/ou parentérale,
et **en ce que** le TTS est prévu pour une application continue et la préparation adaptée pour une administration orale et/ou parentérale est prévue pour une administration discontinue pendant la durée d'application du TTS.

**2.** Utilisation selon la revendication 1, le dérivé d'ergoline étant le lisuride ou l'un de ses sels pharmaceutiquement acceptables.

**3.** Utilisation selon l'une quelconque des revendications 1 à 2, dans laquelle le dérivé d'ergoline a une demi-vie de 0,5 à 4 heures, en particulier, de 1 à 2 heures.

**4.** Utilisation selon l'une quelconque des revendications 1 à 3, dans laquelle le TTS présente une couche de médicament qui présente au moins une matrice contenant un principe actif et/ou un réservoir de principe actif et sur le côté de la peau du réservoir de principe actif, une barrière de diffusion perméable au principe actif.

**5.** Utilisation selon la revendication 4, dans laquelle la matrice et/ou la barrière de diffusion est choisie à condition que l'écoulement transdermique F au travers de la peau humaine soit de l'ordre de 0,1 à 5,0 $\mu g/cm^2/h$.

**6.** Utilisation selon l'une quelconque des revendications 4 à 5, dans laquelle sur le côté détourné de la peau de la matrice et/ou du réservoir de principe actif est disposée une couche de revêtement.

**7.** Utilisation selon l'une quelconque des revendications 4 à 6, dans laquelle la matrice et/ou la barrière de diffusion présente une substance choisie parmi le polyacrylate, le polyuréthane, l'éther de cellulose, la silicone, les composés de polyvinyle, les composés de poly-isobutylène, le silicate et les mélanges de ces substances ainsi que les copolymères de ces composés de polymère, de préférence, le polyacrylate, comme composants principaux de matrice.

**8.** Utilisation selon l'une quelconque des revendications 4 à 7, dans laquelle la barrière de diffusion est un polymère synthétique choisi parmi l'ester de cellulose, l'éther de cellulose, la silicone, la polyoléfine et les mélanges ainsi que les copolymères de ces substances comme composants principaux de barrière.

**9.** Utilisation selon l'une quelconque des revendications 4 à 8, dans laquelle la matrice et/ou le réservoir de principe actif et/ou la barrière de diffusion contiennent un agent de renforcement de la pénétration qui est choisi parmi les alcools aliphatiques en C1 à C8, cyclo-aliphatiques et aromatiques, les alcools gras en C8 à C18 saturés et insaturés, les acides gras en C8 à C18 saturés et insaturés, les hydrocarbures et les mélanges d'hydrocarbures, les esters d'acides gras d'acides gras en C3 à C19 et des alkyl-monools en C1 à C6, les diesters d'acides dicarboxyliques d'acides dicarboxyliques en C4 à C8 et les alkyl-monools en C1 à C6 et les mélanges de ces substances.

**10.** Utilisation selon l'une quelconque des revendications 1 à 9, dans laquelle un jeu de TTS est aménagé, le jeu contenant une multiplicité d'éléments de TTS et dans laquelle les éléments sont aménagés pour administrer des doses différentes.

**11.** Utilisation selon l'une quelconque des revendications 1 à 9, dans laquelle la préparation adaptée pour l'administration orale contient sous forme de comprimés, 25 à 500 $\mu g$ de dérivé d'ergoline par comprimé.

**12.** Utilisation selon l'une quelconque des revendications 1 à 9, dans laquelle la préparation adaptée pour l'administration parentérale sous forme d'une solution pour injection ou pour perfusion contient 25 à 2000 $\mu g$ du dérivé d'ergoline par ml de solution.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9220339 A
- WO 9100746 A
- US 4920989 A